# EUROPEAN PATENT APPLICATION

(11) **EP 4 555 920 A1**
(43) Date of publication of application: **21.05.2025**
(21) Application number: 23210933.0
(22) Date of filing: 20.11.2023
(51) Int. Cl.: A61B 5/00, A61B 5/021, A61B 5/022, A61B 5/024, A61B 5/0225

(54) **A WEARABLE DEVICE FOR CONTINUOUS BLOOD PRESSURE MONITORING**

(71) Applicant: Shiarelax Limited, Sandyford, Dublin, D18 CV48 (IE)
(72) Inventor: BINTING, Malte, 115 50 Stockholm (SE)
(74) Representative: Høiberg P/S

(57) **Abstract**

The invention regards an adhesive patch for oscillatory blood pressure measurements of a subject, the patch comprising: an actuator arranged for application of an external pressure onto a target part of the circulatory system of the subject; a pressure sensor arranged in connection with the actuator, wherein the pressure sensor is adapted to measure the external pressure; wherein the patch is arranged to obtain oscillatory blood pressure measurements of the target part by setting the external pressure applied to the target part above a systolic blood pressure, and thereafter gradually releasing the external pressure until it is below a diastolic blood pressure, while measuring, by the pressure sensor, resulting pressure oscillations of the actuator.

## Description

The present disclosure relates to a wearable device such as an adhesive patch for use in oscillatory blood pressure measurements of a subject.

### Background

Accurate and continuous non-invasive blood pressure monitoring is a key area of research and development, aiming to improve both convenience and reliability over traditional methods. Traditional blood pressure measurement, typically using inflatable cuffs (auscultatory method) or oscillometric devices, provides intermittent readings and may need multiple attempts for accurate results.

Recent advances have led to several innovative, continuous, non-invasive monitoring technologies. These include optical sensors, pulse wave analysis, arterial tonometry, and wearable devices. These emerging technologies address the limitations of traditional cuff-based methods, which are often inconvenient, uncomfortable, and limited in capturing daily blood pressure fluctuations.

Optical sensors, using light to detect blood volume changes, offer continuous monitoring but require calibration with conventional methods. Their cost-effectiveness depends on sensor quality and integration challenges. Pulse wave analysis estimates blood pressure from arterial pulse waveforms, captured by body sensors. Though accurate, it often needs calibration, influencing its cost.

Arterial tonometry, estimating central blood pressure through mathematical models, is integrated into some wearable devices. Its cost varies based on device complexity. Wearable devices, such as smartwatches, use integrated sensors and algorithms for blood pressure estimation. Their accuracy and cost range widely, contingent on their features and validation.

Traditional auscultatory and oscillometric methods, while common, are not suitable for continuous use due to their bulkiness and discomfort. The auscultatory method, considered a gold standard, requires skill to identify Korotkoff sounds, making it subjective and time-consuming.

Oscillometric blood pressure measurements, while commonly used, can be inaccurate due to several factors. The placement and fit of the cuff are critical; incorrect positioning can lead to significant errors in readings. Furthermore, the bulkiness of the cuff makes this method uncomfortable for patients, especially during prolonged or repetitive use.

In addition to discomfort, oscillometric devices can be sensitive to movement, causing inaccuracies in patients who are restless or unable to remain still. The method also tends to overestimate blood pressure in hypotensive patients and underestimate in hypertensive patients, leading to potential misdiagnoses.

Moreover, oscillometric readings can be influenced by external factors such as ambient temperature and arm position relative to the heart. These variables, along with the potential for mechanical failure of the cuff or device, contribute to the overall unreliability of this method for continuous and precise blood pressure monitoring.

Despite significant advancements in continuous, non-invasive monitoring technologies, challenges remain in calibration needs, sensor technology, miniaturization, accuracy, cost, and user-friendliness.

### Summary

Advantageously, the present disclosure addresses the above mentioned challenges by introducing an inexpensive, comfortable, non-invasive device that directly and accurately measures blood pressure continuously.

Therefore, the present disclosure relates to an adhesive patch for oscillatory blood pressure measurements of a subject, the patch comprising:
- an actuator arranged for application of an external pressure onto a target part of the circulatory system of the subject;
- a pressure sensor arranged in connection with the actuator, wherein the pressure sensor is adapted to measure the external pressure;
wherein the patch is arranged to obtain oscillatory blood pressure measurements of the target part by setting the external pressure applied to the target part above a systolic blood pressure, and thereafter gradually releasing the external pressure until it is below a diastolic blood pressure, while measuring, by the pressure sensor, resulting pressure oscillations of the actuator.

In this way, the present disclosure overcomes the disadvantages of the prior art, and can allow for accurate non-invasive continuous blood pressure measurements in a wearable format.

Further, while blood pressure measurement devices of the prior art are difficult to use and prone to user error, for example cuff-based systems such as conventional auscultatory and oscillometric blood pressure measurement devices, the presently disclosed adhesive patch is simple to use and allows for accurate blood pressure measurements without the requirement of being operated by a trained physician.

The simple way of operating the device, including applying it to the skin, results in more accurate measurements, as compared to conventional systems, as user-based measurement errors are prevented. However, in specific examples, In order to obtain the most accurate positioning of the adhesive patch, such as at the target part of the circulatory system, the positioning may be carried out by a healthcare provider, such as a trained physician. Alternatively, or additionally, the adhesive patch may comprise means for providing feedback of the positioning accuracy, thereby allowing for accurate positioning of the adhesive patch also for an inexperienced user of the adhesive patch.

The feedback may for example be provided to the user of the adhesive patch by a remote device, such as a smartphone. The feedback may for example be based on a sensor on a face of an electrical unit of the adhesive patch, arranged to face the user during use. Said sensor may for example be a PPG sensor or another type of optical sensor, alternatively or additionally, the sensor may be an impedance cardiography sensor, a piezoelectric sensor, a capacitive sensor, and/or a bioimpedance sensor.

The presently disclosed adhesive patch can also be arranged such that, once applied to the skin, the adhesive patch will automatically obtain blood pressure measurements by single-sided occlusion of the target part of the circulatory system, acting to further reduce the user interaction, and the measurement errors. Thus the adhesive patch may comprise a sensor that senses that the adhesive patch is accurately positioned. Once the electronic unit of the adhesive patch gets an input that the placement of the adhesive patch is accurate, the electronic unit may be arranged to start the blood pressure measurements.

Further, the adhesive patch is typically arranged to be continuously worn by the user, which is enabled by advantageous properties such as a small size, a small weight and/or a low power consumption. The adhesive patch may further comprise means for communicating with a remote device, typically for transmitting blood pressure measurement data, and/or to provide a user with positioning feedback of the adhesive patch in relation to the target part of the circulatory system. Thus, the user may, in specific examples of the present disclosure, use directions provided on a remote device, such as a smartphone, in order to position the adhesive patch.

This enables accurate blood pressure measurements by a device that is easy to use and comfortable to wear also for longer durations, allowing for accurate and continuous, uninterrupted, blood pressure measurement.

The adhesive patch may be arranged for blood pressure measurements of one or more target parts of the circulatory system, for example by being adapted such that it can be attached to a part of the skin that covers the target part of the circulatory system. For example, the presently disclosed adhesive patch may be arranged so as to cover a target artery of the circulatory system.

The presently disclosed device may for example be arranged so as to be adhered onto the skin, typically a portion of the skin that covers the target part of the circulatory system. The presently disclosed adhesive patch may for example be arranged so as to be adhered to a portion that covers the posterior auricular artery, which is a small artery that arises from the external carotid artery and perfuses the majority of the ear and the scalp posterior to the ear.

The posterior auricular artery is a part of the circulatory system that is arranged in connection with a bone structure, which allows for an externally applied pressure to easily occlude said part of the circulatory system. Thus, in one embodiment of the present disclosure, the target part of the circulatory system is arranged in connection with a bone structure, such as wherein the target part is arranged between the bone structure and the skin.

Thus, the target part may be an artery, and preferably a superficial artery located at, or near, a bone structure. As exemplified above, the target part may arise from the external carotid artery, such as the posterior auricular artery. In such cases, the patch is preferably arranged to adhere to the skin such that it covers at least a part of the posterior auricular artery. In specific examples, the target part is a part of the circulatory system that is, during use of the adhesive patch, located between the actuation part of the adhesive patch and a bone structure of the user. In this way, the target part can be occluded, such as fully occluded, for example by the application of an external pressure by the actuator, that is higher than the systolic blood pressure of the user.

Thus, the adhesive patch can comprise an actuator element adapted to apply a level of compression to targeted part of the circulatory system and/or the surrounding tissue. The actuator may be provided in any format suitable for application of a pressure resulting in said compression. In a specific example of the present disclosure, the actuator consists of, or comprises, a device that generates a pressure by use of an electric field, such as an electroosmotic pump. Said pump may be arranged to apply and/or control the external pressure.

In a further aspect, the present disclosure relates to a blood pressure measurement system comprising: an adhesive patch according to any one of the preceding claims; and a remote device, adapted for data communication with the adhesive patch.

In yet a further aspect, the present disclosure relates to a method of measuring blood pressure comprising: attaching the adhesive patch as disclosed herein to a subject; and measuring blood pressure.

These and other features of the present disclosure will become apparent from the following detailed description, taken in conjunction with the accompanying drawings.

### Description of the drawings

In the following, embodiments and examples will be described in greater detail with reference to the accompanying drawings:
Fig. 1 illustrates an adhesive patch for oscillatory blood pressure measurements of a subject, according to an embodiment of the present disclosure,
Fig. 2 illustrates the connectivity of an adhesive patch for oscillatory blood pressure measurements of a subject,
Fig. 3 illustrates one embodiment of the presently disclosed adhesive patch,
Fig. 4 illustrates one example of the presently disclosure electronic unit, of the adhesive patch,
Fig. 5 illustrates one example of the electronic unit of the adhesive patch from different angles,
Fig. 6 illustrates the interconnections of the components and functionalities of the electronics unit and its connectivities, in one embodiment of the present disclosure.

### Detailed description

Oscillometric blood pressure measurement is widely used in automated devices to determine a person's blood pressure. Typically, the operation includes inflation of a cuff that encases a pressure sensor, to a level high enough to completely obstruct blood flow in the brachial artery. Following this, the cuff undergoes a controlled deflation. As the cuffs pressure decreases, blood starts to flow through the artery once again, generating oscillations in pressure which are detected by the sensor within the cuff.

These conventional devices capture the amplitude of the oscillations throughout the deflation process. It is during this process that it identifies the systolic and diastolic pressures. The systolic pressure, representing the pressure in the arteries during heart contraction, is determined at the point where a sharp increase in the oscillations is noted as the cuff deflates. Meanwhile, the diastolic pressure, which represents the pressure in the arteries when the heart is in a relaxed state, is identified at the point where the oscillations diminish and then cease.

Moreover, the conventional devices utilize the point of maximum oscillation to estimate the mean arterial pressure (MAP). It then calculates the systolic and diastolic pressures using empirical relationships with the mean arterial pressure. These relationships are derived from numerous observations and consider a range of factors including the expected ratios of these pressures to the mean arterial pressure in a general population.

Once calculated, the systolic and diastolic blood pressures are showcased on the device's screen. It is worth noting that modern oscillometric devices incorporate algorithms that can pinpoint errors or irregularities in the measurements, such as an irregular heartbeat or movement artifact, providing warnings or even refusing to display a reading in case of detected errors. Despite its widespread use and convenience, the oscillometric method may not offer the same level of accuracy as auscultatory measurements, which involve the use of a stethoscope and a sphygmomanometer, especially in patients with certain cardiovascular conditions or arrhythmias. Hence, validating the accuracy of any blood pressure measurement technique in individual patients is advised.

Furthermore, as disclosed elsewhere herein, oscillometric blood pressure measurements can be inaccurate due to several factors. The placement and fit of the cuff are critical; incorrect positioning can lead to significant errors in readings. Furthermore, the bulkiness of the cuff makes this method uncomfortable for patients, especially during prolonged or repetitive use.

In addition to discomfort, oscillometric devices can be sensitive to movement, causing inaccuracies in patients who are restless or unable to remain still. The method also tends to overestimate blood pressure in hypotensive patients and underestimate in hypertensive patients, leading to potential misdiagnoses.

Moreover, oscillometric readings can be influenced by external factors such as ambient temperature and arm position relative to the heart. These variables, along with the potential for mechanical failure of the cuff or device, contribute to the overall unreliability of this method for continuous and precise blood pressure monitoring.

The presently disclosed adhesive patch can overcome these drawbacks of conventional oscillometric blood pressure measurements devices. While the adhesive patch is fundamentally different from a conventional oscillometric blood pressure device, there are similarities in the mode of operation. The presently disclosed adhesive patch may for example be arranged to obtain the systolic blood pressure, the diastolic blood pressure and the mean arterial pressure (MAP) by a similar mechanism of occlusion of a target part of the circulatory system. Typically however, the presently disclosed adhesive patch is arranged for single-sided occlusion of the target part of the circulatory system, for example by applying an external pressure onto the target part, and/or a portion of the skin associated with said target part.

### Posterior auricular artery

The posterior auricular artery is a branch of the external carotid artery that supplies blood to the region of the ear and the adjacent area of the scalp. It is located near the ear. After branching off from the external carotid artery, it ascends behind the external acoustic meatus (the ear canal) and crosses over the sternocleidomastoid muscle, which is a muscle that runs along the side of the neck. It then gives off several branches that supply the posterior aspect of the ear and nearby regions of the scalp. It can be found superficial to the mastoid process, which is a bony prominence located behind the ear. It generally travels near the facial nerve and the posterior auricular vein in this region. It's important in various surgical approaches to this region and can sometimes be involved in procedures involving the ear or nearby structures.

In specific examples of the present embodiment, the target part is a part of the external carotid artery, such as the posterior auricular artery.

### MAP

Mean arterial pressure (MAP) is a clinical indicator of perfusion to vital organs. For adults, a MAP of 70 to 110 mm Hg is considered to be normal. Sustaining a MAP within this range ensures that the body's organs receive adequate perfusion. If an individual's MAP is too low, it could indicate insufficient blood flow to the organs, which could potentially lead to organ failure. Conversely, a very high MAP might indicate a significant risk for heart disease or other complications, as it suggests that the blood vessels are under constant high pressure, which could eventually wear out the heart and damage the blood vessels.

The presently disclosed adhesive patch may be arranged such that the external pressure results in a partial or full occlusion of the target part of the circulatory system. Typically, the external pressure is dynamic, such as wherein the external pressure varies over time. For example, at the start of a measurement, the external pressure may initially be set to a value such that the target part is fully occluded, or increased until the target part is fully occluded, such as wherein the pressure exerted on the target part is above the systolic pressure of the target part. Thereafter, the external pressure may be gradually released in a controlled fashion, while the pressure is continuously monitored. At some point, the external pressure is below the diastolic pressure, and the blood flow is returned to the normal state. Throughout the measurement the resulting pulse oscillations are measured and recorded.

The pulse oscillations may for example be used in order to determine blood pressure parameters, such as the systolic pressure, the diastolic pressure and/or the mean arterial pressure (MAP).

As such, the adhesive patch may be arranged to occlude a target part of the circulatory system. The occlusion may be full occlusion of the target part, similar to conventional oscillometric blood pressure measurements, and/or the adhesive patch may be arranged such that the occlusion only partly occludes the target part, for example in order to obtain non-interruptive measurements. Full occlusion of the target part is typically achieved when the external pressure applied to the target part is above the diastolic blood pressure of the target part. Resulting in that the target part is compressed and prevents blood flow.

As mentioned elsewhere in, the adhesive patch may be arranged such that the target part is fully occluded, at least temporarily during measurements. For example, in order to carry out an oscillatory blood pressure measurement similar to conventional oscillometric blood pressure measurements. Alternatively, or additionally, the device may be arranged to carry out blood pressure measurements, by applying a constant external pressure onto the target part. For example, the target part may be partially occluded, typically between the systolic and the diastolic blood pressure. For example, such that the external pressure is in the range between 80-120 mmHg.

In one embodiment of the present disclosure, the target part of the circulatory system is an artery, such as a target artery of the circulatory system. The target artery may for example be the radial artery, the carotid artery and/or the posterior auricular artery. Typically, the artery is arranged such that it can be at least partially occluded, for example by being located between a bone structure and the skin. Thus, application of pressure onto the skin can act to occlude, partially or fully, the target part of the circulatory system.

The target part, such as the target artery, may be a superficial part of the circulatory system, such as a part that is located at, or near, the skin. Such as at a depth of between 0-5 mm from the surface of the skin. Further, the target part may be arranged between a bone structure and the skin. Such as wherein the bone structure is at a depth of maximum 10 mm, more preferably less than 5 mm, and wherein the target part is located at a depth of less than 5 mm, more preferably less than 3 mm.

In one embodiment of the present disclosure, the target part is an artery. Typically, the target part is a small artery and/or an arteriole. Small arteries and arterioles as used herein are smaller branches of arteries that further distribute blood to tissues. Arterioles typically play a crucial role in regulating blood pressure and blood flow. The target part may for example be a part of, or supplied with blood from, the external carotid artery, such as the posterior auricular artery.

In an embodiment of the present disclosure, the adhesive patch is arranged for being adhered to the skin of the subject. The adhesive patch may for example be attached to the skin by mechanical adhesion, such as pressure-sensitive-adhesive. Typically, the patch is arranged for being attached to a neck area, such as behind the ear.

In an embodiment of the present disclosure, the adhesive patch is arranged for being positioned behind the ear of the subject, such that it covers the target part. Typically, the patch is arranged for being attached to the skin such that the patch covers the target part, for example the patch may be arranged to adhere to the skin such that the actuator can apply an external pressure onto the target part, such as the posterior auricular artery.

In one embodiment of the present disclosure, the adhesive patch is arranged to form a waterproof seal against the skin of the subject. The adhesive patch may for example comprise an adhesive layer that is arranged to form a waterproof seal against the skin of the subject, and prevent water from reaching the one or more sensors of a face of the adhesive patch facing the subject, during use. In this way, the electronic unit can be completely sealed off from the surrounding atmosphere. The actuation part of the actuator may for example only be in fluidic connection with the sealed cavity, formed by the adhesive patch and the skin of the user.

The term user, as used herein, is used interchangeably with the term subject, and refers to the person that is the target of the blood pressure measurements and/or a third person that may facilitate the measurements, such as a health professional, an operator, or an individual. The target for the blood pressure measurements are not necessarily the same person as the one who applied the adhesive patch, which operates the remote device, or which analyses and/or diagnoses the measurement results.

In an embodiment of the present disclosure, the adhesive patch comprises an actuator. The actuator may for example be an electroosmotic pump arranged to control the external pressure. An electroosmotic pump operates based on the phenomenon of electroosmosis, which is the motion of liquid induced by an applied electric field across a porous material or through a capillary filled with a fluid containing ions.

The actuator may be any other type of arrangement suitable for applying an external pressure onto the target part of the circulatory system. For example, the actuator may be a pneumatic actuators, a piezoelectric actuators, an electromagnetic actuator, a shape memory alloy (SMA) actuator, a hydraulic actuator, an electroactive polymer (EAP) actuator, and/or a microelectromechanical systems (MEMS) actuator.

A pneumatic actuator may be arranged to inflate and/or deflate a cushion or membrane, using air pressure, which is controlled. Piezoelectric actuators use the piezoelectric effect to create a motion or a force. Piezoelectric actuators are very precise making them able to finely control the external pressure. Electromagnetic actuators use magnetic fields to create force and/or movement. Shape memory alloy (SMA) Actuators change shape in response to environmental changes, such as temperature. They can be used to create actuators that are compact and can exert a controlled pressure when for example electrically stimulated to change temperature. Hydraulic actuators use a liquid medium to transmit force. Electroactive polymer (EAP) actuators rely on materials that change size or shape when stimulated by an electric field. The small size makes them suitable for being used as an actuator of the adhesive patch. Microelectromechanical systems (MEMS) actuators are extremely small devices that can provide precise control at a micro-scale.

In an embodiment of the present disclosure, the actuator is arranged to control the external pressure by adjusting the pressure of an internal volume. The actuator may for example comprise an elastic membrane, or a balloon, that at least partly contains the internal volume. Thus, by adjusting the pressure of the internal volume, the external pressure, that is, during use, applied to the target part of the circulatory system is also adjusted. Similarly, during a measurement, for example wherein the adhesive patch is arranged to gradually decrease the external pressure applied to the target part, the adhesive patch may comprise a pressure sensor that is arranged so as to measure pressure changes to the actuator, such as to the actuation part or the internal volume of the actuation part. The pressure sensor may for example be arranged such that it can measure pressure oscillations of the target part of the circulatory system, such as when the target part is not fully occluded. The measured pressure, such as of the internal volume, may be used in order to derive blood pressure measurements. The blood pressure measurements may be derived, such as calculated, by the adhesive patch itself and/or a remote device that the adhesive patch may be connected to, such as continuously connected to, for example in order to obtain continuous, such as uninterrupted pressure measurements.

In an embodiment of the present disclosure, the actuator comprises an actuation part, arranged to apply the external pressure, and to measure pressure variations in the target part. The actuation part may for example comprise a flexible membrane, or a balloon, arranged to apply the external pressure onto the target part. The flexible membrane may be arranged to contain an internal volume, or may form an internal volume together with the rest of the adhesive patch. The pressure sensor may for example be in fluidic connection with the internal volume. The flexible membrane or balloon, may for example be made of a polymer, such as polydimethylsiloxane, polyethylene, polyurethane, polytetrafluoroethylene, polyvinylidene fluoride or an elastomer such as natural rubber, nitrile rubber or silicone rubber, or a mixture thereof.

The actuation part may be arranged to contain an internal volume of the actuator. The actuation part may for example comprise a membrane or balloon that typically contains, and/or seals, an internal volume of the actuator. The internal volume may be contained by the balloon, the flexible membrane or the flexible membrane together with other components of the adhesive patch. For example, the actuator may comprise an actuator housing that, together with the flexible membrane contains the internal volume. In any event, irrespective of the arrangement of how the internal volume is contained, the flexible membrane is in fluidic connection with the internal volume. Thus, the flexible membrane is affected by pressure changes of the internal volume, and the internal volume is affected by motion of the flexible membrane, such as pulsations of the target part of the circulatory system.

Typically, the adhesive patch is arranged such that the actuation part can, during use, apply an external pressure onto a target area of the circulatory system. The adhesive patch may for example comprise a pressure means arranged for increasing the pressure of the internal volume, such as a pump, for example an electroosmotic pump. Typically, the adhesive patch comprises a pressure sensor. The pressure sensor is preferably arranged to measure the pressure of the internal volume. Thus, pressure changes of the internal volume are measured by the pressure sensor. The measurements of the pressure sensor can thereafter be used to derive blood pressure measurements. The pressure sensor may for example be a piezoresistive pressure sensor, a capacitive pressure sensor, an optical pressure sensor, and/or a mems pressure sensor (micro-electro-mechanical systems).

The adhesive patch is typically arranged such that it can easily be worn by the user at an area of the skin such that measurements of the blood pressure at the target part of the circulatory system can be obtained. For example the adhesive patch may be arranged to be positioned behind the ear. Irrespective of placement, it is a preference that the patch is flexible, in order to conform to the curvature of the skin and in order to withstand movements of the user of the adhesive patch.

In an embodiment of the present disclosure, the size of the patch is smaller than 15 cm², more preferably smaller than 10 cm², yet more preferably smaller than 5 cm². A smaller size may make it easier to wear the adhesive patch and/or to position the adhesive part in difficult areas, for example areas that are exerted to large body movements and/or stretching of the skin. The size of the adhesive patch may for example be between 1 and 15 cm², more preferably between 1 and 10 cm², yet more preferably between 1 and 5 cm².

In an embodiment of the present disclosure, the adhesive patch is stretchable. The adhesive patch typically comprises electronics that form a separate layer of the adhesive patch, while the stretching part of the adhesive part allows for stretching of the adhesive patch.

In an embodiment of the present disclosure, the adhesive patch comprises a sealing layer, arranged to form a sealing barrier to the skin of the user. In this way, the adhesive patch is arranged with a volume between the adhesive patch and the user, that is sealed off from the surrounding atmosphere. This can result in the device being waterproof and/or resistant to dust.

The size of the adhesive patch may be selected so as to make the adhesive patch suitable for the intended use, for example, being positioned such that the adhesive patch covers the target part, or such that the actuator can apply an external pressure onto the target part.

In an embodiment of the present disclosure, the longest dimension of the patch is shorter than 10 cm, more preferably shorter than 7 cm, yet more preferably shorter than 5 cm.

In a specific example of the present disclosure, the adhesive patch has a longest dimension that is in the range of between 10 cm and 2 cm, such as between 8 cm and 3 cm, such as between 6 cm and 4 cm.

In an embodiment of the present disclosure, the length of the patch perpendicular to the longest dimension is shorter than 5 cm, more preferably shorter than 3 cm, yet more preferably shorter than 2 cm.

In an embodiment of the present disclosure, the length of the patch perpendicular to the longest dimension is in the range of between 2 cm and 6 cm, such as between 3 cm and 5 cm.

In specific example of the present disclosure, the adhesive patch has a longest dimension that is in the range of between 10 cm and 2 cm, such as between 8 cm and 3 cm, such as between 6 cm and 4 cm, and at the same time, the length of the patch perpendicular to the longest dimension is in the range of between 2 cm and 6 cm, such as between 3 cm and 5 cm.

In an embodiment of the present disclosure, the longest dimension of the patch is shorter than 10 cm, more preferably shorter than 7 cm, yet more preferably shorter than 5 cm, and at the same time, the length of the patch perpendicular to the longest dimension is shorter than 5 cm, more preferably shorter than 3 cm, yet more preferably shorter than 2 cm.

In an embodiment of the present disclosure, the actuator is arranged to apply the external force onto an area of the subject of between 0.01 (mm)² and 20 (mm)², such as between 0.05 (mm)² and 15 (mm)², such as between 0.5 (mm)² and 12 (mm)², such as around 10 (mm)².

Preferably, the actuator is adapted such that the external force is applied mainly to the target area, and not surrounding areas. Thus, the actuator may be arranged to apply relatively localized force, for example in order to not have an effect on the surrounding tissue.

### Mechanical properties

In an embodiment of the present disclosure, the patch is flexible. A flexible patch may be better to adapt to body movements. As a result, a flexible patch may be better arranged to adhere to the skin, especially during continuous measurements, such as uninterrupted measurements, that may be carried out during a prolonged time period, for example at least 1 hour, but could extend for days. In specific examples, the electronic unit is arranged onto a flexible layer.

In an embodiment of the present disclosure, the patch is substantially planar. The adhesive patch may thus be a planar adhesive patch, that is either generally stiff, or more or less flexible.

In an embodiment of the present disclosure, the patch is substantially non-planar. The adhesive patch may thus be a non-planar adhesive patch, that is either generally stiff, or more or less flexible.

In one embodiment of the present disclosure, the adhesive patch has a shape of a body part. For example, the adhesive patch may have a shape that is suitable for attachment to a part of the neck, such as a part of the neck behind the ear.

### Connectivity

In one embodiment of the present disclosure, the adhesive patch is arranged to record blood pressure data. The adhesive patch may for example comprise a memory, such as a RAM memory, arranged for storing blood pressure data. Alternatively, or additionally, the adhesive patch is arranged to communicate with a remote device, as disclosed elsewhere herein. The adhesive patch may, in such cases, be arranged to continuously stream blood pressure data to the external device. Thus, derivation, such as calculation, of the blood pressure and/or blood pressure parameters such as systolic blood pressure (SBP), diastolic blood pressure (DBP), pulse pressure, ankle-brachial index, ambulatory blood pressure monitoring average, and/or mean arterial pressure (MAP) may be conducted by the adhesive patch itself, or by a remote device. In such an instance, the adhesive patch may be arranged to transmit blood pressure data continuously during a measurement, or the adhesive patch may be arranged to transmit the blood pressure data at a specific event, such as when a user requests, on a remote device, that the adhesive patch is to transmit data. In other examples, the adhesive patch may be arranged to transmit at a specific interval, such as once per day, and/or when the adhesive patch is in the vicinity of the remote device.

Pulse Pressure (PP) is the difference between the systolic and diastolic pressure. It's an indicator of the health and tone of the arterial walls. A normal pulse pressure is typically about 40 mmHg, though this can vary based on individual factors.

Ankle-Brachial Index (ABI) is a ratio of the blood pressure in the lower legs to the blood pressure in the arms. Differences in this ratio can indicate peripheral arterial disease.

Ambulatory Blood Pressure Monitoring (ABPM) Average is the average blood pressure recorded over a 24-hour period. It provides a more accurate reflection of blood pressure changes throughout the day and night.

In any event, the adhesive patch may be arranged to either transmit the measured blood pressure data, and/or derive blood pressure parameters in case those have been derived by the adhesive patch itself. In one example, the adhesive patch is arranged to record blood pressure data comprising the measured pressure oscillations.

In one embodiment of the present disclosure, the patch is arranged to transmit, or transfer, blood pressure data and/or derived blood pressure parameters, to an external device, such as a processing device, including a smartphone, a tablet, and/or a computer.

In specific examples, the blood pressure data may comprise information on the positioning of the adhesive patch. For example, whether the actuator is accurately positioned with respect to the target part. In such an instance, the adhesive patch may be arranged to provide feedback on the positioning to the user, such as through a remote device. The adhesive patch may for example be arranged to provide feedback on how the user should reposition the device in order to be accurately positioned with respect to the target part of the circulatory system.

In a specific example of the present disclosure, the blood pressure data comprise information on how the patch should be repositioned in order to be accurately positioned on the subject, such as with respect to the target part of the circulatory system.The adhesive patch may for example be arranged to present blood pressure data on a remote device. Thus, the adhesive patch may form a system for blood pressure measurements, together with a remote device.

### Sensors

In an embodiment of the present disclosure, the adhesive patch comprises an ECG sensor, arranged for obtaining ECG data. The adhesive patch may for example comprise a first ECG sensor/electrode arranged on a face of the electronic unit. The adhesive patch may for example comprise a second ECG sensor/electrode arranged to be positioned remotely to the electrical unit, for example wherein the second electrode is in electrical contact with the electrical unit by a connector, such as electrical wiring.

An ECG (Electrocardiogram) sensor, as used herein, is a medical device used to record the electrical activity of the heart over a period of time. It utilizes electrodes placed on the skin to detect the electrical signals generated by the heart muscle during each heartbeat. The data may for example be used to diagnose irregular heart rhythms, ischemia, and other cardiac conditions.

In an embodiment of the present disclosure, the adhesive patch is arranged to derive one or more ECG leads, such as one lead, two leads, three leads, or even more, such as 12 and/or wherein the leads include none, a single, or a plurality of virtual leads.

The ECG sensor may for example comprise two or more ECG electrodes.

In one example, the ECG sensor is a two lead ECG sensor.

A two-lead ECG sensor refers to an ECG setup where two electrodes are placed on the patient's body to record the electrical activity of the heart. A two-lead ECG effectively has one recording channel, meaning it records the difference in electrical potential between two points (or electrodes) on the body.

The electrodes can be placed at different locations on the body, commonly on the limbs or chest. The choice of location can affect the information gathered by the ECG.

In an embodiment of the present disclosure, the ECG is arranged to derive ECG data. In specific examples the ECG data comprise heart rate and/or heart rate variability measurements. The adhesive patch, or the remote device, may be arranged to take the ECG data into account when deriving the blood pressure parameters.

In an embodiment of the present disclosure, the adhesive patch comprises a PPG-sensor. A Photoplethysmogram (PPG) sensor is a bio-optical device designed to non-invasively measure the volume change of blood vessels in the microvascular bed of tissue through the detection of variations in light absorption caused by cardiovascular pulsations. The sensor operates by emitting a light, commonly in the visible or infrared spectrum, onto the skin, whereupon a portion of the light is absorbed by the blood vessels and the remainder is reflected back to the sensor's photodetector. The adhesive patch, or the remote device, may be arranged to take the PPG measurements into account when deriving the blood pressure parameters.

In an embodiment of the present disclosure, the adhesive patch comprises a temperature sensor. A temperature sensor, as used herein, is a device that detects and measures heat to determine the temperature of the environment or an object, such as the user. It works by translating the physical property of temperature into a quantity that can be measured, usually an electrical signal. The adhesive patch, or the remote device, may be arranged to take said electrical signal into account when deriving the blood pressure parameters. There are various types of temperature sensors, such as thermocouples, thermistors, infrared sensors, and semiconductor sensors, each operating on different principles to measure temperature. These sensors find applications in a wide range of fields including industrial settings, medical devices, household appliances, and automotive systems, offering an essential tool for monitoring and controlling temperature in diverse environments.

In an embodiment of the present disclosure, the adhesive patch comprises an internal motion sensor, such as an inertial measurement unit (IMU). An IMU is a type of sensor that measures and/or records a body's specific force, angular rate, and sometimes the orientation of the body, using a combination of accelerometers, gyroscopes, and sometimes magnetometers. The IMU may for example be arranged to detect the position of the user, for example whether the user is lying down.

### Software

In an embodiment of the present disclosure, the adhesive patch comprises a processing unit and a memory, and wherein the memory comprises instructions that, when executed by the processor, causes the adhesive patch to obtain oscillatory blood pressure measurements. The adhesive patch may for example comprise instructions such that the patch obtains blood pressure measurements by the method as disclosed elsewhere herein.

### Method

The adhesive patch may be arranged to obtain blood pressure measurements data and/or blood pressure parameters in one or more ways. For example, the adhesive patch may be arranged to obtain said information in a similar manner as a conventional oscillometric blood pressure device, wherein a pressure is applied to occlude part of the circulatory system, and thereafter gradually release the applied pressure while monitoring the pressure response, conventionally done by monitoring the pressure of the cuff. Alternatively, or additionally, the adhesive patch may be arranged to obtain said information by applying an external pressure onto a target part of the circulatory system.

In an example the adhesive patch is arranged to obtain oscillatory blood pressure measurements in a process similar to conventional oscillatory blood pressure measurements. The adhesive patch may for example be arranged to gradually increase the pressure applied onto a target part of the circulatory system, such as of the user. At the same time, the adhesive patch may be arranged to measure the applied pressure, such as the external pressure. The adhesive patch may be arranged to increase the pressure until the applied pressure is above the systolic blood pressure of the target part, which may for example be identified by the absence of pressure oscillations in pressure measurements, by the pressure sensor.

Following, increasing of the pressure above the systolic pressure of the target part, the adhesive patch may be arranged to gradually decrease the applied pressure and simultaneously measure, by the pressure sensor, resulting pressure oscillations of the target part.

In this way, blood pressure data may be obtained, such as wherein the blood pressure data comprises or consists of pressure measurements of the pressure sensor, such as wherein the blood pressure data comprises pressure oscillations. Furthermore, the blood pressure data may be used to derive, such as calculate, blood pressure parameters.

Alternatively, or additionally, the adhesive patch can be arranged to obtain blood pressure data, measurements by applying a constant applied pressure. This may be used in order to obtain oscillatory blood pressure measurements.

For example, the adhesive patch may be arranged such as to set the pressure that is applied onto the target part at a predetermined level, such as in between the systolic and diastolic pressure of the target part.

Thereafter, the adhesive patch may be arranged so as to obtain blood pressure measurements by the pressure sensor, such as pressure oscillations. The pressure oscillations are typically obtained when the applied pressure is set such that the external pressure applied to the target part is between the diastolic and the systolic blood pressure of the target part.

### Calculations

In an embodiment of the present disclosure, the patch is arranged to obtain, such as calculate, blood pressure and/or blood pressure parameters such as SBP, DBP and/or MAP from the obtained blood pressure measurements, such as from the blood pressure data. In specific examples, the adhesive patch is arranged for obtaining, such as calculating blood pressure and/or blood pressure parameters such as SBP, DBP and/or MAP from pressure oscillations.

In an embodiment of the present disclosure, the adhesive patch comprises a temperature sensor. The temperature sensor may for example be arranged to measure a body temperature, such as of the user.

An elevated body temperature, such as during fever of hyperthermia, may result in vasodilation and/or an increased heart rate.

An increased body temperature often leads to vasodilation, where the blood vessels expand. This can lead to a decrease in blood pressure because the blood has a larger area to flow through, reducing the force against the vessel walls.

Similarly, a fever or a higher body temperature due to other reasons, can lead to an increased heart rate, which could potentially increase the blood pressure temporarily due to the heightened cardiac output.

Similarly, hypothermia can lead to vasoconstriction, where the blood vessels narrow. This generally increases blood pressure because the blood has a smaller area to flow through, increasing the force against the vessel walls. Hypothermia also often results in a decreased heart rate, which could potentially lower the blood pressure due to the reduced cardiac output.

Further, individuals with certain health conditions might respond differently to changes in body temperature, with potentially more significant fluctuations in blood pressure. Another factor is the use of medications, which can also influence how body temperature affects blood pressure.

The adhesive patch, and/or the remote device may be arranged to take the body temperature into account and/or other factors as mentioned herein, such as the heart rate, PPG measurements, and/or body position, for obtaining, such as calculating, the blood pressure and/or the blood pressure parameters.

In an embodiment of the present disclosure, the patch is arranged to obtain, such as calculate, the blood pressure and/or the blood pressure parameters, from the resulting pressure oscillations by different mathematical models, depending on temperature measurements of the temperature sensor. Thus, the body temperature of the user of the adhesive patch may have an effect on how the blood pressure and/or blood pressure parameters are determined.

In an embodiment of the present disclosure, the patch is arranged to adjust the external pressure based on temperature measurements of the temperature sensor.

In an embodiment of the present disclosure, the subject is a human or an animal, such as a mammal. The term subject is used interchangeably with the term user herein, and refers to an operator of the adhesive patch and/or a bearer of the adhesive patch, and/or an operator of a remote device in communication with the adhesive patch.

In an embodiment of the present disclosure, adhesive patch is arranged for continuous blood pressure measurements.

In an embodiment of the present disclosure, the adhesive patch is arranged for continuous, non-interrupted, blood pressure measurements. The adhesive patch may for example be arranged so as to measure, continuously, without interruption, blood pressure of a subject. The adhesive patch may for example be arranged to apply an external pressure onto the target part of the circulatory system that is in the range of from the systolic pressure to the diastolic pressure of said target part.

### Detailed description of drawings

The invention will in the following be described in greater detail with reference to the accompanying drawings. The drawings are exemplary and are intended to illustrate some of the features of the present disclosure, and are not to be construed as limiting to the presently disclosed invention.

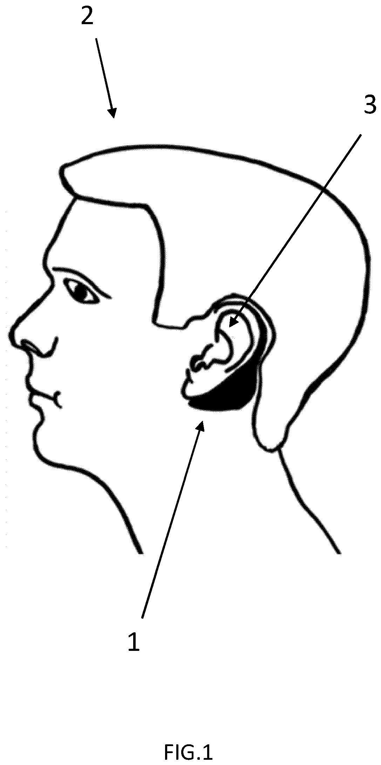

Fig. 1 illustrates an adhesive patch (1) for oscillatory blood pressure measurements of a subject (2), according to an embodiment of the present disclosure. The adhesive patch can be arranged such that it is to be located, during use, behind the ear of the subject. For example, in such a way that a sensing area of the actuator at least partly overlaps with a target part of the circulatory system, as disclosed elsewhere herein.

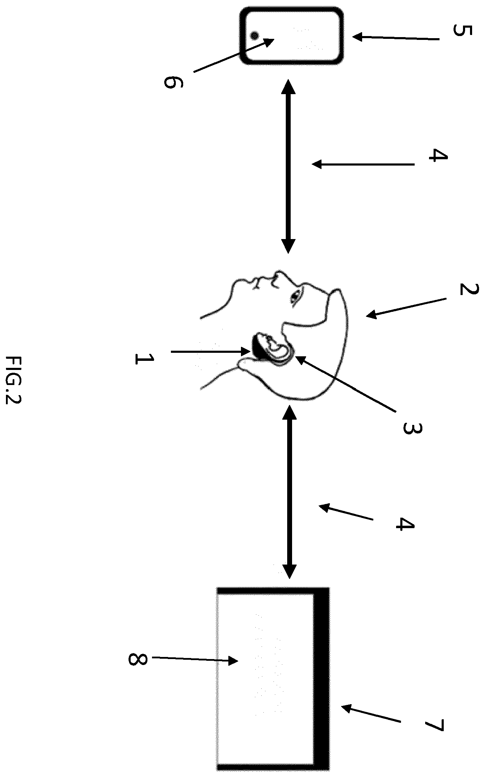

Fig. 2 illustrates the connectivity of an adhesive patch (1) for oscillatory blood pressure measurements of a subject (2). While, the adhesive patch may comprise an I/O interface access for wired communication, the adhesive patch may alternatively or additionally be configured for wireless communication (4) with a remote device (5) and/or with a remote server. The remote device may for example be a computer, a laptop, a tablet and/or a mobile phone, such as a smartphone. The remote device may be arranged to execute a mobile application, through which the subject and/or another person, can monitor and/or configure the adhesive patch. For example, the remote device may be arranged to display blood pressure measurements in this way. Further, the remote device may be arranged such that it allows for access to real-time blood pressure measurements, historical blood pressure measurements, analysis data of blood pressure measurements, measurement data of the temperature sensors and/or of the motion sensors. Further the remote device may be arranged, together with the adhesive patch to provide feedback, such as feedback of the positioning of the adhesive patch, and/or feedback of the measurement procedure. For example, the remote device may be arranged to provide feedback in case the adhesive patch and/or the remote device identified factors affecting measurement accuracy, this may for example include the activity of the user, the positioning of the user, and/or error messages of the device. In this way, the remote device may be arranged to provide feedback to the user in order to optimize the measurement accuracy.

The adhesive patch (1) is typically designed to establish a robust connection with the remote server (7), primarily through wireless communication technologies. This design offers flexibility in connectivity. In some configurations, the adhesive patch interfaces with the remote server via a dedicated remote device, which may, as such, act as a bridge, relaying data. The remote device could for example be a smartphone, a tablet, or a specialized medical device, enabling a versatile and user-friendly approach to data transmission.

Alternatively, the adhesive patch may be configured for direct communication with the remote server. This may for example be achieved through an integrated communication module within the patch itself. The communication module may comprise means for connecting to the internet. This direct connection not only streamlines data transfer but also enhances the reliability of the communication, ensuring that vital health data is consistently and accurately relayed to the server.

The remote server may be arranged to be accessed by a web application (8), for example a data management application. The application may for example be designed to be accessible by healthcare professionals, and as such, to provide them with a comprehensive dashboard or interface where they can monitor real-time blood pressure readings. The server may comprise analytics tools that can process the blood pressure measurement data, and thereby offer insights into trends, anomalies, and patterns in the patient's blood pressure. This gives the healthcare professionals a deeper understanding of the patient's condition, facilitating more accurate and timely diagnosis.

Furthermore, the server can be configured to send alerts or notifications to healthcare professionals in case of abnormal readings. This feature is crucial for immediate intervention in emergency situations, potentially saving lives by allowing prompt medical response.

Additionally, the remote server can be integrated with electronic health record (EHR) systems, enabling a seamless flow of information. This integration ensures that the patient's blood pressure data becomes a part of their comprehensive medical record, accessible by authorized healthcare providers across different healthcare settings.

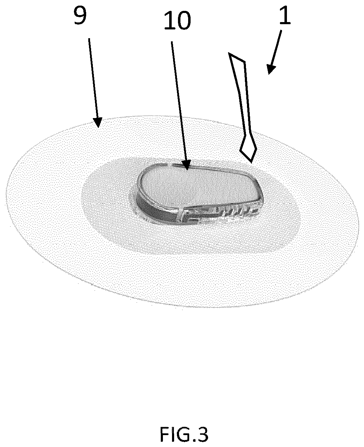

Fig. 3 illustrates one embodiment of the presently disclosed adhesive patch (1). The adhesive patch may comprise, as an integral component, an electronic unit (10). The electronic unit may be arranged to for example obtain blood pressure data and/or to transmit said data to a remote device. The adhesive patch is typically arranged to adhere to the skin of the subject. For example, the adhesive patch may be provided with a sealing layer (9). In one example, the face of the sealing layer that is arranged to face the subject during use, is provided with an adhesive. The adhesive may for example be biocompatible, hypoallergenic, skin-friendly, and capable of maintaining a secure bond without causing irritation or discomfort to the subject. In specific examples, the adhesive may for example be an acrylic adhesive, a silicon-based adhesive, a hydrocolloid adhesive, a hydrogel adhesive and/or a pressure-sensitive adhesive.

Acrylic adhesives are in general utilized for their strong adhesion, suitable for medical use and ensuring a reliable bond with the skin. Silicone-based adhesives are chosen for their gentle adherence, particularly beneficial for sensitive skin, and offer the advantage of easy repositioning and removal. Hydrocolloid adhesives typically provide a cushioning effect due to the gel-forming agents, enhancing comfort during prolonged wear. Hydrogel adhesives have a high moisture content which may be advantageous for sensitive skin areas. Further, pressure-sensitive adhesives (PSAs) offer customizable adhesion strengths, and can be arranged to maintain the adhesiveness during prolonged wear.

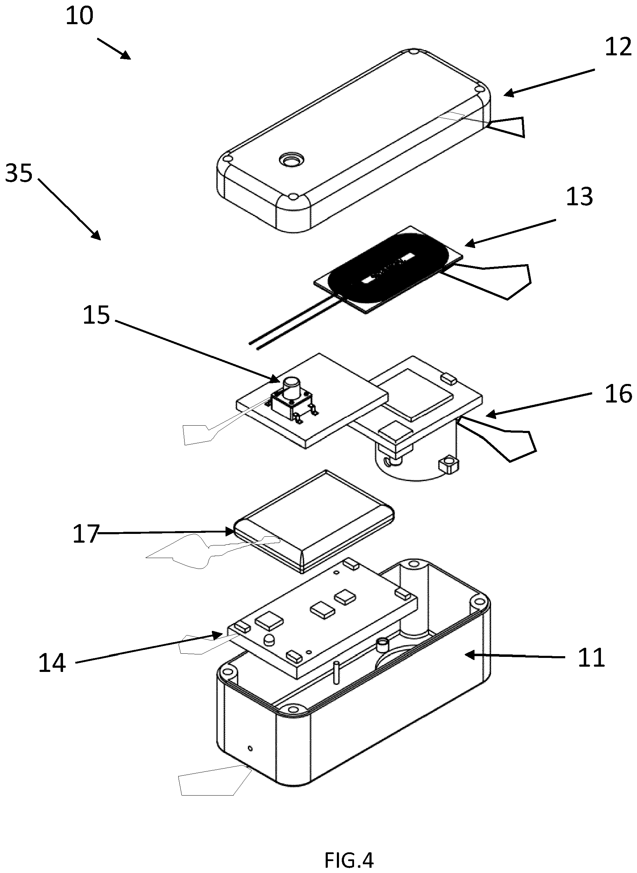

Fig. 4 illustrates one example of the presently disclosed electronic unit (10), of the adhesive patch (1). The electronic unit may be provided with an adhesive layer, in order to adhere to the subject during use. The electronic typically comprises a casing (11) having a casing lid (12) thereby forming an encasing for containing components of the electronic unit (10). The electronic unit may further comprise a wireless charging means (13) for example a coil through which the electronic unit can be wirelessly charged. The wireless charging means may be arranged for inductive coupling or capacitive coupling.The electronic unit may further comprise a control unit (14), for example a PCT having electronic components, for example arranged to control the electronic unit. An interaction means (15), for example a push-button, is provided and may be used to activate the adhesive patch, such as to power on the adhesive device. Further, the interaction means may be used to activate wireless pairing with the remote device, and/or to start measurements. The electrical unit (35) may comprise the wireless charging means (13), the control unit (14), the interaction means (15), the actuator (16), and the power source (17).

Furthermore, the actuator (16) is arranged towards the subject during use. A power source (17), for example a battery, is arranged to provide power to the electronic unit, and may further be powered by the wireless charging means (13).

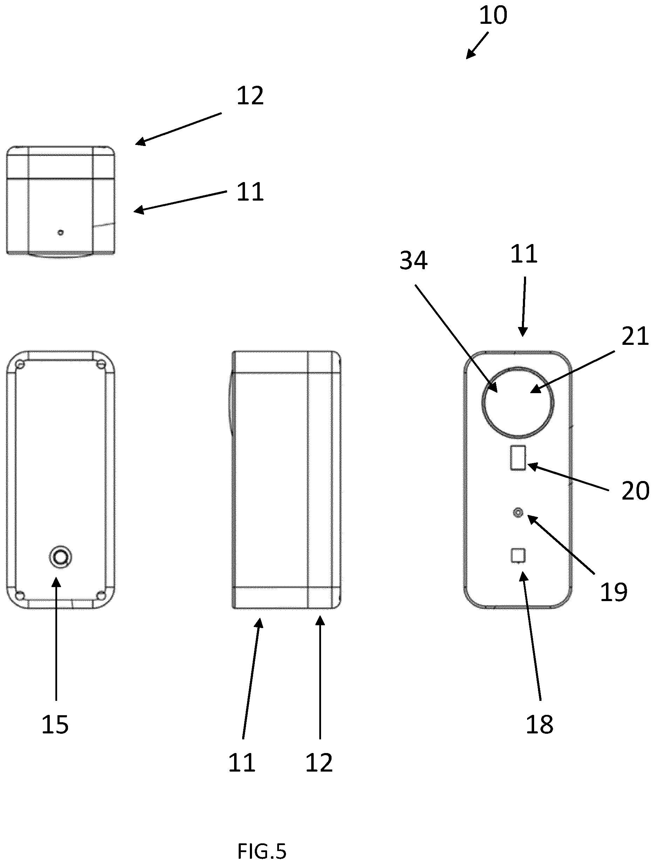

Fig. 5 illustrates one example of the electronic unit (10) of the adhesive patch (1) from different angles. The electronic unit (10) comprising a casing (11) having a casing lid (12). A first face of the electronic unit comprises an opening for the interaction means (15), thereby facilitating control of the electronic unit. Futhere, a second face, typically arranged on an opposite side from the first face, may comprise a plurality of openings. Thereby facilitating measurements of the subject. The second face may comprise a temperature sensor (18), a first ECG electrode (19), one or more optical sensors (20), such as an oxygenometer and/or a PPG sensor, and an actuation part (21) of the actuator (16). The electronic unit may further comprise a second ECG electrode that is arranged to be connected at a position distal to the casing of the electronic unit, and may be electronically connected to the electronic unit by a conductive wire.

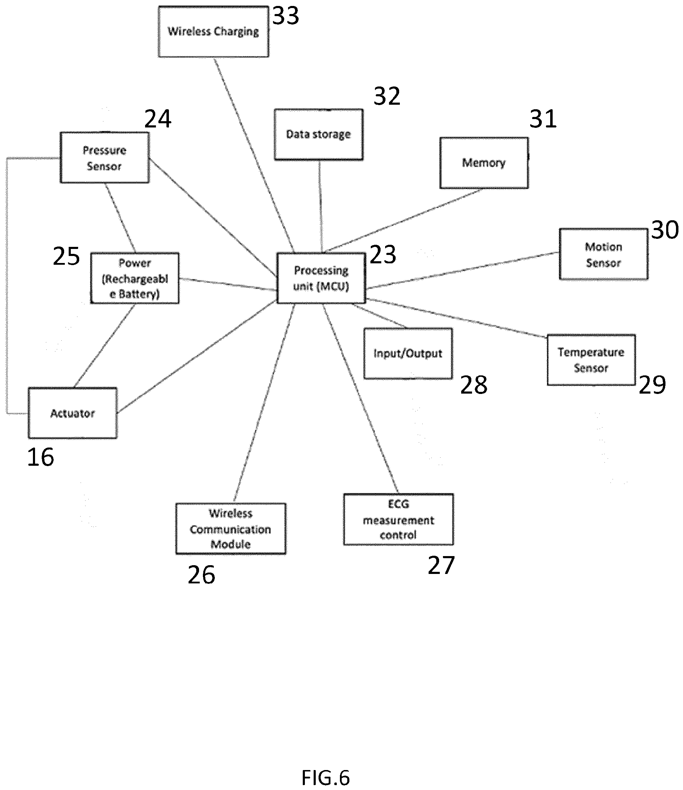

Fig. 6 illustrates the interconnections of the components and functionalities of the electronics unit and its connectivities, in one embodiment of the present disclosure.

At the center of the system lies the Microcontroller Unit (23, MCU), which serves as the central processing unit orchestrating the operations of the device.

Connected to the MCU is a pressure sensor (24), responsible for capturing the blood pressure data. The MCU also interfaces with a power source (25), ensuring a steady supply of energy to the various components. The MCU further controls the actuator (16), typically such that the actuator is arranged to both apply a certain pressure level to a target area, and further to relay pressure to the pressure sensor.

Further expanding the device's capabilities, the MCU is connected to a wireless communication module (26), which may be arranged for facilitating seamless data transmission and/or remote monitoring. For comprehensive health tracking, an ECG measurement control (27) is also linked to the MCU. This allows the device to obtain ECG measurements, providing a more holistic view of the user's cardiovascular health.

The device further comprises an input/output interface (28) that is in connection with the MCU, allowing for programmable access to the electronics unit, and to monitor and to configure the electronics unt.. This feature allows for customizations and updates to be easily implemented.

In addition, the MCU may further be connected to a suite of sensors, increasing the device's functionality and accuracy. A temperature sensor (29) and a motion sensor (30) can be integrated, offering insights into environmental and user activity factors that might influence blood pressure readings. To support data management, the MCU is linked to both a memory module (31) and potentially also a separate data storage unit (32), ensuring ample space for recording and preserving vital health data.

Lastly, the device includes a mechanism for wireless charging (33), illustrating a commitment to user convenience and modern technology integration. This feature allows for easy and efficient recharging of the device, maintaining its readiness for continuous health monitoring.

### Numbering

1. Adhesive patch
2. Subject
3. Ear
4. Wireless communication
5. Remote device
6. Mobile Application
7. Remote Server
8. Web application
9. Sealing layer
10. Electronic unit
11. Casing
12. Casing lid
13. Wireless charging means
14. Control unit
15. Interaction means
16. Actuator
17. Power source
18. Temperature sensor
19. ECG Electrode
20. Optical sensor
21. actuation part
22. Target part
23. Microcontroller Unit
24. Pressure sensor
25. Power source
26. Wireless communication module
27. ECG measurement control
28. I/O interface
29. Temperature sensor
30. Motion sensor
31. Memory
32. Data storage
33. Wireless charging
34. Actuation part
35. Electrical unit

### Items

1. An adhesive patch for oscillatory blood pressure measurements of a subject, the patch comprising:
   - an actuator arranged for application of an external pressure onto a target part of the circulatory system of the subject;
   - a pressure sensor arranged in connection with the actuator, wherein the pressure sensor is adapted to measure the external pressure;
   wherein the patch is arranged to obtain oscillatory blood pressure measurements of the target part by setting the external pressure applied to the target part above a systolic blood pressure, and thereafter gradually releasing the external pressure until it is below a diastolic blood pressure, while measuring, by the pressure sensor, resulting pressure oscillations of the actuator.
2. The adhesive patch according to any one of the preceding items, wherein the patch is arranged for being adhered to the skin of the subject.
3. The adhesive patch according to any one of the preceding items, wherein the patch is arranged for being positioned behind the ear of the subject, such that it covers the target part.
4. The adhesive patch according to any one of the preceding items, wherein the adhesive patch is arranged to form a waterproof seal against the skin of the subject.
5. The adhesive patch according to any one of the preceding items, wherein the target part is a target artery.
6. The adhesive patch according to item 5, wherein the target artery is a superficial artery located at, or near, a bone structure, such that the artery may be occluded.
7. The adhesive patch according to any one of items 5-6, wherein the target artery is, or arises from, the external carotid artery.
8. The adhesive patch according to any one of items 5-7, wherein the target artery is the posterior auricular artery.
9. The adhesive patch according to any one of the preceding items, wherein the actuator comprises an electroosmotic pump arranged to control the external pressure.
10. The adhesive patch according to any one of the preceding items, wherein the actuator controls the external pressure by adjusting the pressure of an internal volume of the actuator.
11. The adhesive patch according to item 10, wherein the actuator comprises an actuation part, such as a flexible membrane or a balloon, that is arranged to apply the external pressure onto the target part, and wherein the actuation part is in fluidic connection with the internal volume.
12. The adhesive patch according to any one of items 10-11, wherein the pressure sensor is arranged to measure the pressure of the internal volume, such as wherein the pressure sensor is arranged within, or exterior to, the internal volume.
13. The adhesive patch according to any of the proceeding items, wherein the size of the patch is smaller than 15 cm², more preferably smaller than 10 cm², yet more preferably smaller than 5 cm².
14. The adhesive patch according to any of the proceeding items, wherein the longest dimension of the patch is shorter than 10 cm, more preferably shorter than 7 cm, yet more preferably shorter than 5 cm.
15. The adhesive patch according to any of the proceeding items, wherein the length of the patch perpendicular to the longest dimension is shorter than 5 cm, more preferably shorter than 3 cm, yet more preferably shorter than 2 cm.
16. The adhesive patch according to any of the proceeding items, wherein the actuator is arranged to apply the external force onto an area of the subject of between 0.01 (mm)² and 20 (mm)², such as between 0.05 (mm)² and 15 (mm)², such as between 0.5 (mm)² and 12 (mm)², such as around 10 (mm)².
17. The adhesive patch according to any of the proceeding items, wherein the patch is flexible.
18. The adhesive patch according to any of the proceeding items, wherein the patch is substantially planar.
19. The adhesive patch according to any of the proceeding items, wherein the patch is arranged to record blood pressure data comprising the measured pressure oscillations.
20. The adhesive patch according to item 19, wherein the patch is arranged to transfer blood pressure data to an external device, such as a processing device, including a smartphone, a tablet, and/or a computer.
21. The adhesive patch according to any of the proceeding items, wherein the blood pressure data comprise information on whether the actuator is accurately positioned onto the target part.
22. The adhesive patch according to any of the proceeding items, wherein the blood pressure data comprise information on how the patch should be repositioned in order to be accurately positioned on the subject.
23. The adhesive patch according to any of the proceeding items, wherein said patch comprises an ECG sensor, arranged for obtaining ECG data.
24. The adhesive patch according to item 23, wherein the ECG comprises two ECG leads.
25. The adhesive patch according to any one of items 23-24, wherein the ECG data comprises heart rate and/or heart rate variability measurements.
26. The adhesive patch according to any one of the preceding items, wherein the adhesive patch comprises a PPG-sensor.
27. The adhesive patch according to any one of the preceding items, wherein the adhesive patch comprises a temperature sensor.
28. The adhesive patch according to any one of items 23-27, wherein the adhesive patch comprises an internal motion sensor, such as an internal measurement unit (IMU).
29. The adhesive patch according to any of the proceeding items, wherein the patch comprises a processing unit and a memory, and wherein the memory comprises instructions that, when executed by the processor, causes the adhesive patch to obtain oscillatory blood pressure measurements.
30. The adhesive patch according to any one of the preceding items, wherein the patch is arranged to obtain oscillatory blood pressure measurements by:
   - gradually increasing the pressure applied onto the target part, and simultaneously measure the applied pressure, until the applied pressure is above the systolic blood pressure of the target part; and thereafter
   - gradually decreasing the applied pressure and simultaneously measuring, by the pressure sensor, resulting pressure oscillations of the target part.
31. The adhesive patch according to any of the proceeding items, wherein the patch is arranged to obtain oscillatory blood pressure measurements by:
   - setting the pressure applied onto the target part at a predetermined pressure; and
   - measuring the resulting pressure oscillations.
32. The adhesive patch according to any of the proceeding items, wherein the patch is arranged to calculate blood pressure data from the resulting pressure oscillations.
33. The adhesive patch according to any one of items, wherein the patch comprises a temperature sensor.
34. The adhesive patch according to item 33, wherein the patch is arranged to calculate the blood pressure data from the resulting pressure oscillations by different mathematical models, based on temperature measurements of the temperature sensor.
35. The adhesive patch according to any one of items 33-34, wherein the patch is arranged to adjust the external pressure based on temperature measurements of the temperature sensor.
36. The adhesive patch according to any of the proceeding items, wherein the subject is a human or an animal, such as a mammal.
37. The adhesive patch according to any of the proceeding items, wherein the wearable device is arranged for continuous blood pressure measurements.
38. The adhesive patch according to any of the proceeding items, wherein the wearable device is arranged for continuous, non-interrupted, blood pressure measurements.
39. A blood pressure measurement system comprising:
   - an adhesive patch according to any one of the preceding items;
   - a remote device, adapted for data communication with the adhesive patch.
40. A method of measuring blood pressure comprising:
   - attaching the adhesive patch of any one of item 1-38 to a subject;and
   - measuring blood pressure.

## Claims

1. An adhesive patch for oscillatory blood pressure measurements of a subject, the patch comprising:
• an actuator arranged for application of an external pressure onto a target part of the circulatory system of the subject;
• a pressure sensor arranged in connection with the actuator, wherein the pressure sensor is adapted to measure the external pressure;
wherein the patch is arranged to obtain oscillatory blood pressure measurements of the target part by setting the external pressure applied to the target part above a systolic blood pressure, and thereafter gradually releasing the external pressure until it is below a diastolic blood pressure, while measuring, by the pressure sensor, resulting pressure oscillations of the actuator.

2. The adhesive patch according to claim 1, wherein the patch is arranged for being adhered to the skin of the subject.

3. The adhesive patch according to any one of the preceding claims, wherein the adhesive patch is arranged to form a waterproof seal against the skin of the subject.

4. The adhesive patch according to any one of the preceding claims, wherein the target artery is the posterior auricular artery.

5. The adhesive patch according to any one of the preceding claims, wherein the actuator comprises an electroosmotic pump arranged to control the external pressure.

6. The adhesive patch according to any one of the preceding claims, wherein the actuator controls the external pressure by adjusting the pressure of an internal volume of the actuator.

7. The adhesive patch according to claim 6, wherein the actuator comprises an actuation part, such as a flexible membrane or a balloon, that comprises or is in fluidic connection with the internal volume, and wherein the actuation part is arranged to apply the external pressure onto the target part.

8. The adhesive patch according to any one of claims 6-7, wherein the pressure sensor is arranged to measure the pressure of the internal volume, such as wherein the pressure sensor is arranged within, or exterior to, the internal volume.

9. The adhesive patch according to any of the proceeding claims, wherein the adhesive patch is arranged to obtain blood pressure data, and wherein the blood pressure data comprises information of whether the adhesive patch is accurately positioned onto the target part.

10. The adhesive patch according to claim 9, wherein the adhesive patch is arranged to provide the subject with information, such as via a remote device, of how the adhesive patch should be repositioned in order to be accurately positioned onto the target part.

11. The adhesive patch according to any of the proceeding claims, wherein said patch comprises at least one further sensor, such as an ECG sensor, arranged for obtaining ECG data, a temperature sensor, and/or an inertial measurement unit, and wherein the adhesive patch is arranged to derive blood pressure values based on the pressure oscillation and measurement data obtained from the at least one further sensor.

12. The adhesive patch according to claim 11, wherein the adhesive patch comprises the ECG sensor and wherein the adhesive patch comprises a first ECG electrode arranged on a first face of an electronic unit of the adhesive patch, and a second ECG electrode arranged for being remotely located from the electronic unit and connected to said electronic unit by a connector.

13. The adhesive patch according to any one of the preceding claims, wherein the patch is arranged to obtain repetitive oscillatory blood pressure measurements, wherein the measurements are separated by a first timeperiod, and wherein the first timeperiod is decreased if the blood pressure is outside of a normal blood pressure interval, and increased if the blood pressure is within the normal blood pressure interval, such as 120/80 mmHg.

14. A blood pressure measurement system comprising:
• an adhesive patch according to any one of the preceding claims;
• a remote device, adapted for data communication with the adhesive patch.

15. A method of measuring blood pressure comprising:
• attaching the adhesive patch of any one of claim 1-13 to a subject;and
• measuring blood pressure.
